# EUROPEAN PATENT SPECIFICATION

(11) **EP 4 374 156 B1**
(45) Date of publication and mention of the grant of the patent: **04.03.2026**
(21) Application number: 21766254.3
(22) Date of filing: 22.07.2021
(51) Int. Cl.: G01N 15/14, G01N 15/10

(54) **METHOD AND SYSTEM FOR MONITORING CELL SETTLING**
VERFAHREN UND SYSTEM ZUR ÜBERWACHUNG DER ZELLABSETZUNG
PROCÉDÉ ET SYSTÈME DE SURVEILLANCE DE SÉDIMENTATION DE CELLULES

(43) Date of publication of application: 29.05.2024
(60) Divisional application: 25163715.3
(73) Proprietor: Sartorius BioAnalytical Instruments, Inc., Bohemia, NY 11716 (US)
(72) Inventor: OLSZOWY, Michael, Arvada, CO 80004 (US); REIF, Oscar-Werner, 37079 Göttingen (DE); KENNINGTON, Aaron, Ann Arbor, MI 48108 (US)
(74) Representative: Novagraaf International SA
(86) International application number: PCT/US2021/042832
(87) International publication number: WO 2023/003561

(56) References cited:
- EP-A1- 2 450 706
- EP-A1- 2 803 998
- EP-A1- 3 663 760
- US-A1- 2007 072 301

## Description

### BACKGROUND

Cells (e.g., cultured cells, explanted tissue samples) can be incubated in a variety of media and exposed to a variety of conditions (e.g., temperature, dissolved gas levels, radiation, humidity, added substances, electrical or magnetic fields, viruses, microorganisms) in order to assess the response of the cells to the applied conditions. The response of the cells to the applied conditions can be measured in order to assess the efficacy of a drug or treatment, to assess the toxicity of a substance (e.g., of an experimental drug or treatment), to investigate the effect of a genetic modification of the cells, to investigate the metabolomics, structure, or other properties of the cells and/or tissue formed therefrom, or to determine some other information. This assessment can include extracting a small volume of the sample for analysis via flow cytometry, as an example. However, automatically performing such an analysis for a large number of samples of a multi-well sample plate (e.g., a 96-well or larger sample plate) can take sufficient time that cells in the samples may settle, the samples may exhibit significant evaporative fluid loss, or the samples may exhibit some other change(s), such as changes in pH, dissolved oxygen, osmolarity, for example, resulting in unwanted effect on cell health, cell counts or other data generated by the flow cytometer or other analysis of the samples. In high-throughput flow cytometry many such multi-well sample plates may be queued for sequential analysis using the same flow cytometry system. Individual plates may sit in stasis for up to 48 hours or more between being removed from an incubator, placed in the queue, and ultimately sampled. In such a scenario, the above effects on the samples (e.g., settling, evaporation, etc.) may be exacerbated.

### SUMMARY

An aspect of the present invention relates to a method for detecting a consistency of a biological sample, as defined in claim 1.

Yet another aspect of the present invention relates to a non-transitory computer-readable medium, as defined in claim 13.

Yet another aspect of the present invention relates to a system for detecting a consistency of a biological sample, as defined in claim 14.

Preferred features of the invention are set out in the dependent claims.

### BRIEF DESCRIPTION OF THE FIGURES

Figure 1A depicts a sample containing cells during a first period of time, according to an example embodiment.
Figure 1B depicts the sample of Figure 1A during a second, subsequent period of time, according to an example embodiment.
Figure 2A depicts a sample containing cells and indicator beads during a first period of time, according to an example embodiment.
Figure 2B depicts the sample of Figure 2A during a second, subsequent period of time, according to an example embodiment.
Figure 3A depicts flow cytometry particle counts generated by extracting a volume of the sample of Figure 2A, according to an example embodiment.
Figure 3B depicts flow cytometry particle counts generated by extracting a volume of the sample of Figure 2B, according to an example embodiment.
Figure 4 depicts elements of an automated flow cytometry system, according to an example embodiment.
Figure 5 is a flowchart of an example method for detecting and monitoring and/or remediating cell settling and/or evaporation in a multi-well sample plate with indicator beads included within the sample.
Figure 6 is a flowchart of an example method for detecting and monitoring and/or remediating cell settling and/or evaporation in a multi-well sample plate with indicator beads included in sentinel wells separate from cell sample wells.
Figure 7 is a flowchart of an example method for detecting cell settling and/or evaporation in a multi-well sample plate.
Figure 8 shows experimental results assessing the evaporation of water over time from multi-well sample plates.
Figure 9 shows experimental results assessing the evaporation of water over time from multi-well sample plates.
Figure 10 shows experimental results assessing the evaporation of water over time from sealed and non-sealed multi-well sample plates.

### DETAILED DESCRIPTION

### I. Overview

EP 2 803 998 A1 discloses a method for quantifying cells of interest that are potentially contained in a blood-derived sample, such as circulating tumour cells (CTCs), and a method for evaluating reliability of the system for quantifying the cells of interest.

It is desirable in a variety of applications to measure and analyze the behavior of a large number of tissue and/or cell samples (e.g., dozens, hundreds, or thousands of samples representing variation across a variety of experimental conditions). Such analysis can facilitate the assessment of a variety of therapeutic treatments, environmental conditions, genetic modifications, communicable diseases, or other specified conditions in order to assess the efficacy of a treatment, to determine the toxicity of a substance, or to determine some other information of interest. For example, such high-throughput screening of hundreds or thousands of different samples can facilitate the identification of high-growth clone cell lines, while reducing the cost and/or time associated with such processes. This detection and analysis can include extracting small volumes from each sample to perform flow cytometry and generating cell counts or other information about the population(s) of cells in each sample, such as cell health and consistency of the sample.

A robotic sample handling apparatus can be used to reduce the cost and/or time required to perform such assessments on many samples. Such "high throughput screening" apparatus and methods can include using multi-well sample plates or other multi-sample containers that permit robots or other apparatus to interact with dozens, hundreds, or thousands of samples in a single container in an automated manner. This can include placing a multi-well sample plate into an automated flow cytometry instrument that includes a robotic sample extraction head. Such a sample extraction head can move in two or more dimensions, facilitating the extraction of fluid from samples across the multi-well sample plate, as well as extracting chemical reagents, flushing of cleaning fluids, or other substances from reservoirs located nearby. The iQue^{®} screeners, high throughput screening, volumetric flow cytometry systems, are examples of such a robotic sample handling apparatus that can provide fast, low-cost screening of samples.

An additional robotic arm or other automated sample handling apparatus can also be used to present multiple different multi-well sample plates to the flow cytometry instrument over time, facilitating the automated assessment of even more samples with no or minimal human intervention. Such a system can operate to screen very many samples over an extended period of time without human intervention, e.g., for longer than 48 hours. Such a combined system can include expanded reservoirs for rinse fluids, cleaning fluids, or other consumables. Additionally or alternatively, local reservoirs that are accessible by a sample head of a robotic sample handling apparatus (e.g., one of the iQue^{®} series of systems) can be augmented by additional remote reservoirs that are operable to automatically replenish the local reservoirs. The QMax^{®} Refill Module is an example of such a system that is compatible with the iQue^{®} screener.

While such high throughput, automated sample handling and assessment systems may allow for reduced time to assess a given number of samples, that time is not reduced to zero. Long queues of sample plates in towers may be waiting for analysis up to 48 hours, for example. In turn, over these prolonged periods of time, assessment of a first sample in a multi-well plate versus an assessment of the last sample in the same multi-well plate may vary drastically due to experimentally significant changes to the cellular environment. For example, the cellular environment may be subject to evaporation and chemical changes over time, such as changes in pH, dissolved oxygen, carbon dioxide, glucose concentrations, osmolarity, and other measures that might affect cell physiology, like the formation of reactive oxygen species and infection. Additionally, there may be physical changes, such as cell settling.

These processes, occurring over such extended periods of time, can result in the generation of cell counts that are inaccurate in both an absolute sense (e.g., relative to the true count of cells in a sample) as well as relative to counts of other cell types in a sample. Such inaccuracies can be due to evaporation of water or other fluids in the sample and/or due to different rates of settling between different cells types, which can be related to differences in the range, average, and/or distribution of the specific gravity or other properties of cells in each of the different cell types. Thus, depending on the exact location within a sample from which fluid is drawn for analysis, one type of cell may be over-represented in the analysis (e.g., due to not having settled as much) while another type may be under-represented (e.g., due to having settled to a location in the sample below the location from which fluid is drawn).

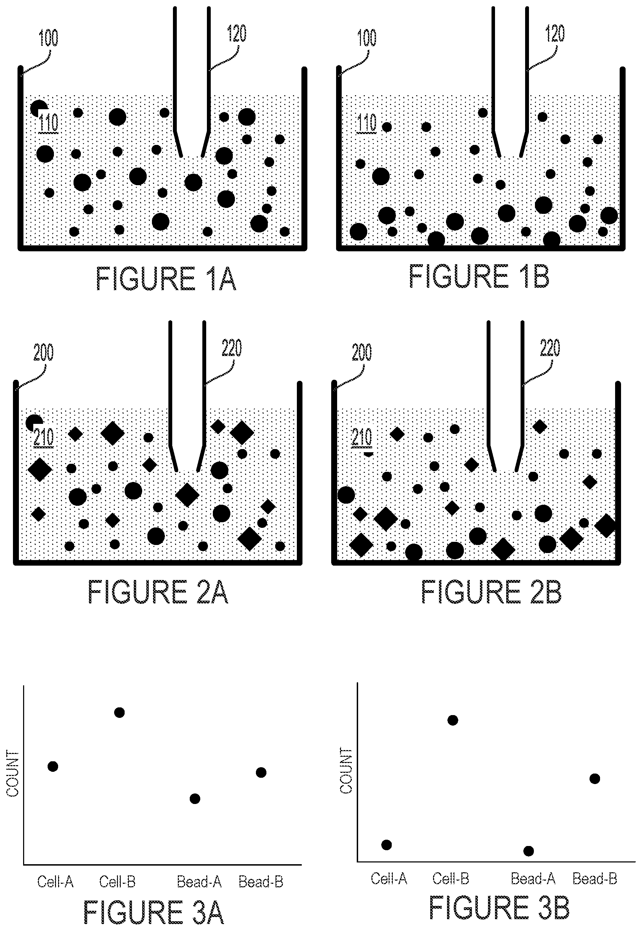

Figures 1A and 1B illustrate exemplary settling processes for a sample 110 before a period of settling and after the period of settling, respectively. The sample 110 is contained within a container 100 (e.g., a well of a multi-well sample plate). The sample 110 includes a first population of cells (small circles) and a second population of cells (large circles) disposed within a medium that may include liquids (e.g., real or simulated extracellular fluid, a fluid phase of a cell growth medium, etc.), solids (e.g., additional populations of cells, calibration beads, solid textured surfaces to facilitate growth of cells thereon), or other substances (e.g., a gel comprising fibrin, collagen, or other proteins or other macromolecules suspended within or otherwise interspersed by saline or some other liquid). A probe 120 extends into the sample 110 and can be used to extract a volume of the sample 110 for flow cytometry and/or some other analysis or assessment.

At the time period depicted in Figure 1A, the two cell populations are roughly equally dispersed throughout the volume of the container 100. Thus, a volume of the sample 110 extracted via the probe 120, when analyzed, is likely to result in cell counts for each of the two cell populations that are accurate in an absolute sense (e.g., relative to the volume of the sample 110 extracted therefrom) and relative to each other.

However, the cells can exhibit settling or other processes that result in cells exhibiting a non-uniform or otherwise undesired distribution throughout the container 100. Additionally or alternatively, evaporation or other processes may result in the detected concentration of the cells, as detected using a volumetric flow cytometry apparatus (e.g., an ique^{®} system), being inaccurate. The extent of such settling, evaporation, or other processes may be related to the amount of time that the sample is exposed to a non-controlled environment, e.g., while dozens or hundreds of other samples of a multi-well sample plate that includes the container 100 are assessed by an automated flow cytometer.

Figure 1B depicts the sample 110 of Figure 1A following such a period of settling. As shown, the second cells (larger circles) have exhibited a significant degree of settling such that the second cells are mostly located near the bottom of the container 100. In contrast, the first cells (smaller circles) have exhibited less settling, still being relatively uniformly distributed throughout the container 100. Thus, a volume of the sample 110 extracted via the probe 120 would lead to inaccurate counts of both the first and second cells within the sample 110, on an individual level (with the first cells being mis-counted to a lesser degree compared to the second, larger cells) and relative to each other (the counted ratio between the first and second cells would favor the first cells to a greater degree than the true ratio represented in the sample 110).

It is desirable to detect such settling, evaporation, or other processes in order to remediate those processes and/or to account for the effects of such processes on measured cell counts in subsequent analyses. This can include storing the detected cell counts for a sample along with a flag or other annotation indicating that the counts were detected from a sample that exhibited more than a specified amount of settling, evaporation, or other processes that can cause the counts to be inaccurate. Additionally or alternatively, the cell counts can be corrected, based on some detected aspect of the degree of settling, evaporation, or other processes. Detection that a sample exhibits more than a specified amount of settling, evaporation, or other processes can be indicated to a user via an audio, visual, or other type of alert. This can allow the user to take remediating actions, e.g., to manually shake or otherwise re-homogenize or resuspend the sample, to add fluids to rehydrate the sample, to terminate the screening process, or to take some other action. Such an alert can be accompanied by halting or slowing the screening process to allow such actions to be taken and/or for a user to command the system to resume screening without taking such processes. Detection that a sample exhibits more than a specified amount of settling, evaporation, or other processes can also result in some automatic remediation to resuspend the sample, thereby reversing any settling that may have occurred.

One solution to automatically remedy the settling of sample contents is to resuspend the sample to re-distribute the contents throughout the sample. This can include shaking the sample by, for example, vibrating a sample (e.g., vibrating a multi-well sample plate that contains the sample) for some specified period of time (e.g., at least 15 seconds). Additionally or alternatively, a circular shaking motion can be applied to the sample using, e.g., an orbital shaker. In yet another example, a probe or paddle can be inserted into the sample and moved around in order to stir or mix the sample. In yet another example, contents of a sample can be repeatedly aspirated into and then expelled from a needle or other probe so as to mix or otherwise resuspend the sample contents.

However, repeatedly performing such a resuspension step (e.g., after assessing each sample, after a pre-specified period of time, or according to some other set protocol) increases the amount of time necessary to assess a set number of samples. Additionally, each instance of resuspension results in a perturbation to the sample, which may result in apoptosis or other processes that lead to changes in cell counts or other parameters of interest of a sample. Yet further, resuspending the samples according to a specified protocol does not guarantee that the samples are acceptably non-settled or otherwise resuspended when sampled.

Accordingly, one solution to the above problems is to include one or more populations of indicator beads in the sample and/or in a 'sentinel' or control well that is in the same multi-well sample plate as one or more samples of interest. A known amount of such indicator beads can be added to a sample and/or to a sentinel well so as to facilitate detection of settling and/or evaporation or otherwise quantifying the 'consistency' of the samples. For example, settling and/or evaporation can be detected by using a volumetric flow cytometry apparatus (e.g., an iQue^{®} system) to detect the absolute concentration of the indicator beads in a sample by detecting both the count of such beads in an amount sampled from the well as along with the volume (total, or as a rate over time) of the sampled amount. The consistency of the samples (e.g., the degree of settling and/or evaporation) can then be determined based on a deviation between the observed count of the indicator beads from an expected count. That the sample's consistency deviates from an expected consistency (e.g., that the sample has experienced more than a specified acceptable amount of settling and/or evaporation) can then be determined if the observed count differs from the expected count by more than a specified threshold amount (absolute, or ratio), or if some other quantification of the sample consistency deviates from an expected consistency by more than a threshold value according to some other calculation.

In some examples, more than one population of indicator beads can be added that differ with respect to specific gravity or other properties (e.g., diameter, size, geometry, surface properties like hydrophobicity, oleophobicity, texture, roughness, porosity) such that they exhibit differential settling. A known amount (absolute, or relative to each other) of such indicator beads can be added to a sample and/or to a sentinel well so as to facilitate detection of settling or other factors related to the consistency of the sample and/or the sentinel well. For example, the consistency (e.g., degree of settling) can be detected by determining a deviation between expected ratio(s) between the one or more populations of indicator beads and the observed ratio(s) between the different populations determined from volumes of fluid extracted from a sample and/or sentinel well. Such an assessment of the degree of settling or other factor(s) related to the sample consistency can be accomplished even when using a non-volumetric flow cytometry apparatus, based on the ratio of the observed counts of the two (or more) different populations of indicator beads.

To enhance the detection of settling or other processes acting on different cell types of interest within a sample that affect the sample's consistency, the specific gravities (or diameters, sizes, geometries, surface properties, or other properties) of the different population(s) of indicator beads can be selected to approximate the properties of the different corresponding types or populations of cells. For example, the specific gravity of a first population of indicator beads can be within a range of specific gravities exhibited by a first population of cells in a sample and the specific gravity of a second population of indicator beads can be within a range of specific gravities exhibited by a second population of cells in the sample.

Figures 2A and 2B illustrate such settling processes for a sample 210 before a period of settling and after the period of settling, respectively. The sample 210 is contained within a container 200 (e.g., a well of a multi-well sample plate). The sample 210 includes a first population of cells (small circles), a second population of cells (large circles), a first population of indicator beads (small diamonds), and a second population of indicator beads (large diamonds) disposed within a medium. The medium may include liquids (e.g., real or simulated extracellular fluid, a fluid phase of a cell growth medium, etc.), solids (e.g., additional populations of cells, calibration beads, solid textured surfaces to facilitate growth of cells thereon), or other substances (e.g., a gel comprising fibrin, collagen, or other proteins or other macromolecules suspended within or otherwise interspersed by saline or some other liquid). A probe 220 extends into the sample 210 and can be used to extract a volume of the sample 210 for flow cytometry and/or some other analysis or assessment.

At the time period depicted in Figure 2A, the two cell populations and the two indicator bead populations are roughly equally dispersed throughout the volume of the container 200. Thus, a volume of the sample 210 extracted via the probe 220, when analyzed, is likely to result in counts for each of the two cell populations and for each of the two indicator bead populations that are accurate in an absolute sense (e.g., relative to the volume of the sample 210 extracted therefrom) and relative to each other. Figure 3A illustrates example counts for the cell populations ("Cell-A" and "Cell-B") and for the indicator bead populations ("Bead-A" and "Bead-B") that can be generated by analyzing a volume of the sample 200 extracted via the probe 220 at the time illustrated in Figure 2A

However, the cells and/or the indicator beads can exhibit settling or other processes that result in the cells and/or the indicator beads exhibiting a non-uniform or otherwise undesired distribution throughout the container 200. The extent of such settling or other processes may be related to the amount of time that the sample is exposed to a non-controlled environment, e.g., while dozens or hundreds of other samples of a multi-well sample plate that includes the container 200 are assessed by an automated flow cytometer.

Figure 2B depicts the sample 210 of Figure 2A following such a period of settling. As shown, the second cells (larger circles) and the second indicator beads (larger diamonds) have exhibited a significant degree of settling such that the second cells and the second indicator beads are mostly located near the bottom of the container 200. In contrast, the first cells (smaller circles) and the first indicator beads (smaller diamonds) have exhibited less settling, still being relatively uniformly distributed throughout the container 200. Thus, a volume of the sample 210 extracted via the probe 220 would lead to inaccurate counts of both the first and second cell populations and the first and second indicator bead populations within the sample 210, on an individual level (with the first cells and the first indicator beads being mis-counted to a lesser degree) and relative to each other (the counted ratio between the first and second indicator beads/cells would favor the first indicator beads/cells to a greater degree than the true ratio represented in the sample 210). Figure 3B illustrates examples of such misleading counts for the cell populations ("Cell-A" and "Cell-B") and for the indicator bead populations ("Bead-A" and "Bead-B") that can be generated by analyzing a volume of the sample 200 extracted via the probe 220 at the time illustrated in Figure 2B.

As noted above, the specific gravities or other properties of the one (or more) populations can differ to facilitate detection of settling and/or evaporation of the contents of a sample. This can include the specific gravities (or other properties) of the indicator beads being selected to correspond to the specific gravities (or other properties) that are characteristic of one or more cell types of interest. For example, the first indicator beads of Figures 2A and 2B can have a specific gravity that is within a range of specific gravities that is characteristic of the first cells of Figures 2A and 2B, and the second indicator beads of Figures 2A and 2B can have a specific gravity that is within a range of specific gravities that is characteristic of the second cells of Figures 2A and 2B.

Determining the degree of settling and/or evaporation exhibited by a sample can include comparing the observed absolute value of the count and/or volumetric concentration of one or more populations of indicator beads, or a ratio between two such counts, to a threshold value. A sub-threshold (or super-threshold) value can indicate that the amount of settling and/or evaporation has exceeded an acceptable level. This detection can be recorded along with the counts, or can be used to correct the observed counts to more accurately reflect, based on the observed counts of the indicator bead population(s), what the likely 'true' counts would have been, had the level of settling and/or evaporation been less.

Additionally, or alternatively, such detection can be used to determine that some remedial action should be taken. This can include automatically shaking or otherwise resuspending the sample to re-distribute the cells and indicator beads within the sample, disregarding one or more previously-measured cell counts, generating an alert for a user, halting further screening, etc.

Detecting that the amount of settling and/or evaporation has exceeded an acceptable level can take a variety of forms. For example, a ratio between measured counts of first and second indicator beads in a sample, or between the measured and expected counts of a single population of indicator beads in a sample, can be determined and compared to an expected ratio for that sample (e.g., a ratio of the amounts of the first and second indicator beads that were initially added to the sample). The ratio being less than a specified threshold of the expected ratio (e.g., less than 0.8 of the expected ratio, less than 0.95 of the expected ratio) can be used as an indicator that the sample has experienced a threshold amount of settling and that the sample should be resuspended, that an alert should be generated, that screening should halt, that an annotation should be recorded for the sample, that the measured counts should be adjusted to account for the observed degree of settling and/or evaporation, or that some other remedial and/or analytical action should be taken. The specified threshold can be set according to an amount of settling and/or evaporation that is acceptable. So, if more settling and/or evaporation is acceptable for a particular application, the threshold can be set lower (e.g., 0.8, 0.7), but if less settling and/or evaporation is acceptable for the application, the threshold can be set higher (e.g., 0.9, 0.95, 0.99).

Note that the ratios above can be determined in either direction (e.g., a count of first indicator beads divided by a count of second indicator beads OR a count of second indicator beads divided by a count of first indicator beads). Additionally, or alternatively, the ratio can be determined in a single direction, and the ratio then compared to two threshold levels in order to determine whether settling and/or evaporation has occurred. So, if it is unacceptable for the ratio in either directed to fall below 0.8 of an expected ratio, then the ratio in a single direction can be determined and settling and/or evaporation detected if the single ratio is less than 0.8 or greater than 1.25. In another example, if it is unacceptable for the ratio in either direction to fall below 0.9 of an expected ratio, then the ratio in a single direction can be determined and settling and/or evaporation detected if the single ratio is less than 0.9 or greater than 1.11.

Further, detection that the amount of settling and/or evaporation has exceeded an acceptable level can occur at discrete points in time (e.g., after pulling a specified volume from a sample well) or continuously. For example, the counts of two different indicator beads from a sample can be continuously updated and the determination as to settling and/or evaporation can be made as soon as the ratio between real-time, continuously updated counts moves below (or above) a specified threshold value (assuming that more than a minimum number of total counts or minimum sample time or volume has been reached). Such continuous, real-time detection can be implemented as a "gate," along with other analysis-related gates assessed by the flow cytometry system (e.g., gates related to forward scatter, side scatter, marker expression, or combinations of multiple factors or inter-relationships thereof) to control the operation of and/or analyses generated by the flow cytometry system.

As noted above, when an unacceptable level of settling is detected in a sample, the sample can be automatically resuspended (e.g., shaken) to re-distribute the cells, indicator beads, or other contents of the sample(s). For example, a multi-well sample plate containing the sample can be vibrated for a period of time, e.g., 15 seconds. An automated flow cytometry system can then proceed to assess further samples. In some examples, the system can go back and re-assess the sample that originally resulted in the notification of unacceptable levels of settling. This can be done in order to obtain, for that sample, counts that are not affected by the detected unacceptable level of settling. Additionally or altematively, this can be done to ensure that the resuspension has resulted in redistribution of the sample contents. For example, the original sample can be re-assessed and another determination made as to the level of settling present.

If the level of settling is less than a threshold level (e.g., a different, more stringent level than the original threshold, in order to ensure that the resuspension has been effective in substantially re-distributing the cells, indicator beads, and other contents of the sample), the system can proceed to assess further samples. If the level of settling has not been sufficiently reduced, other remedial actions can be taken. This can include halting screening, generating an alert, applying further resuspension, applying resuspension of a different type or degree (e.g., for a longer period of time, at a different magnitude, at a different frequency or RPM), or other actions. Generating an alert can include providing one or more of a variety of audio, visual, tactile, or other varieties of alert via a computer, flow cytometer user interface, cellphone, pager, or other medium. Such an alert could be provided as part of a display of data generated by a flow cytometer, e.g., as a color coding, asterisk, note, annotation, flag, exclamation point, or other indication provide with (e.g., overlaid on) a display of cell counts or other data generated about a well or other sample(s) within a multi-well sample plate.

Indeed, such additional resuspension can be done in order to adaptively determine the correct duration, magnitude, frequency or RPM, or other properties of the resuspension to ensure that the contents of the samples are sufficiently re-distributed. For example, if 15 seconds of shaking is insufficient to fully re-distribute the sample contents, but an additional 5 seconds was sufficient, then a single period of shaking having a duration of 20 seconds can be applied the next time that an unacceptable level of settling is detected. Other remedial actions in response to the shaking being insufficient to bring the level of settling down to an acceptable level can include providing an alert that human intervention is needed, assessing a different sample to assess the level of settling (e.g., to account for 'bad' samples that do not comport with the expected levels of indicator beads, that have 'solidified', or that are otherwise unresponsive to resuspension), or some other remedial action(s).

Additionally or altematively, the detected counts and/or ratios between two or more types of indicator beads can be used to adjust cell counts for corresponding cell types without having to resuspend or otherwise agitate the sample. This can be done, e.g., where the samples are sensitive to shaking or other available methods of resuspension. This can also be done where the settling properties (e.g., specific gravity) of a type of indicator bead is specified to match the corresponding settling properties of an associated cell type. In such examples, the degree of deviation of the indicator bead count from an expected value for a sample (e.g., a count of the indicator beads that was initially added to the sample) can be used to adjust the count for an associated cell type to correct for the presumably similar degree of settling between the indicator beads and the cells of the cell type and/or to correct for the degree of evaporation of the sample, which will affect both counts equally. For example, if the observed count for a type of indicator bead is 20% less than expected, then the count of the corresponding cell type can be adjusted upward by 25%, to correct for an assumed similar magnitude of settling by cells of the cell type and/or to correct for the degree of evaporation experienced by the sample that contains both the indicator beads and the cells.

As described above, one or more populations of indicator beads can be added to a sample in order to detect that an amount of settling and/or evaporation has occurred in that sample and/or to quantitatively correct for the effects of such settling and/or evaporation on the observed counts for associated cell types in the sample. However, in some examples it is desirable to omit such indicator beads from the sample. This can be done, e.g., to avoid confounding effects of the beads on the behavior of cells in the sample, to facilitate identification and counting of cells in the sample (e.g., in examples wherein the cells of interest are difficult to distinguish from the available types of indicator beads), or to provide some other benefit or avoid some other unwanted effect. In such examples, where the indicator beads are not disposed within "production" samples containing cells or other biological contents of interest, the two or more populations of indicator beads may instead be located in one or more "sentinel wells" or "control wells" on the same multi-well sample plate as the "production" sample-containing wells.

These sentinel wells can contain sentinel samples that include the indicator beads as well as other contents to cause the settling behavior of the indicator beads and/or the evaporative behavior of fluids in the sentinel well to approximate the behavior they would exhibit were they disposed in "production" samples with the cells of interest. In some examples, this can include creating "sentinel" samples in the substantially the same manner as used to generate "production" samples but adding the indicator beads only to the "sentinel" samples at some point. Additionally, the "production" samples and the "sentinel" samples can be prepared using the same or similar growth medium, introducing the same or similar cells into both types of samples, and then incubating both types of samples using the same or similar incubation parameters or equipment.

Assessment of a multi-well sample plate that includes such sentinel wells can thus proceed by extracting and assessing samples from the "production" wells (e.g., using flow cytometry to generate cells counts of one or more types of cells in each well), interspersed periodically by extracting and assessing samples in one or more sentinel wells of the same multi-well sample plate. The assessment of the sentinel well volume can then be used, as described above, to assess the level of settling and/or evaporation in the sentinel well and, by inference, the level of settling and/or evaporation in the other wells, including the "production" wells, of the multi-well sample plate.

Actions or analysis taken as a result of the assessment of the sentinel well (e.g., as a result of determining, based on the volume extracted from the sentinel well, that an unacceptable level of settling and/or evaporation has occurred in samples of the multi-well sample plate) may be the same or similar to those described above in connection with "production" samples that also contain indicator beads. For example, a determination that an unacceptable level of settling has occurred (due, e.g., to a ratio between first and second indicator bead populations being less than a threshold level) can result in resuspension of the multi-well sample plate, followed by re-assessment of the sentinel well and/or one or more "production" wells assessed prior to the assessment of the sentinel well, use of the detected pre- resuspension indicator bead counts to adjust or otherwise correct corresponding cell counts from one or more "production" wells assessed prior to the assessment of the sentinel well, and/or some other remedial action(s) performed in response to the determination that the sentinel well is exhibited an unacceptable level of settling and/or evaporation. Additionally or alternatively, an alert can be generated, screening can be halted, an annotation related to the sample(s) on the plate can be recorded, counts for one or more other samples on the plate can be corrected based on the count(s) of indicator beads observed from the sentinel well, or other actions or analysis can occur.

The assessment of such sentinel well(s) of a multi-well sample plate can be performed on a set protocol or according to some other consideration. For example, a sentinel well can be assessed once every n "production" wells (e.g., five "production" wells can be assessed followed by assessment of the sentinel well), once every set duration of time (e.g., "production" wells are assessed until a duration of five minutes has passed, at which point a sentinel well is assessed), once the earlier of a number of wells has been assessed or a set duration of time has elapsed, or according to some other set protocol. The parameters of such a protocol can be adaptively set in order to reduce the amount of resuspension that is applied to a multi-well sample plate (e.g., in order to reduce the time it takes to assess every well of the plate, in order to reduce the amount of resuspension-related agitation that the samples of the plate experience). For example, upon the completion of a timer, the sentinel well can be assessed. If the sentinel well exhibits an unacceptable degree of settling, the plate can be resuspended, the timer reset, and the duration of the timer can be reduced so as to next assess the sentinel well earlier with respect to the settling process in the samples. If, instead, the sentinel well exhibits an acceptable degree of settling, the duration of the un-reset timer can be increased. The magnitude of any increase or decrease of such a timer can be pre-set, or can be determined based, e.g., on a magnitude of the degree of settling detected in the sentinel well.

Note that, while reference is made throughout to systems wherein a plurality of different biological samples are contained within multi-well sample plates, the systems and methods described herein can be applied to other multi-sample contexts. For example, a plurality of samples can be contained within discrete sample containers (e.g., vials, tubes, cuvettes, etc.) configured to be placed within a tray, rack, centrifuge carousel, or other multi-sample container. Such multiple samples, contained within respective discrete sample containers, can then be assessed using an automated flow cytometry apparatus as described herein. When the methods described herein result in the determination that the consistency of one or more of the samples deviates by more than a specified threshold amount from an expected consistency (e.g., the concentration of an indicator bead population deviates from an expected concentration by more than a threshold amount, or a ratio between counts of two different indicator bead populations deviates from an expected ratio by more than a threshold amount), then such samples can be resuspended, e.g., by shaking the tray or container containing the plurality of discrete sample containers. Additionally or alternatively, an alert can be generated, one or more cell counts can be corrected, the sampling apparatus can stop, an annotation can59 be made in a database of cell counts, or some other action can be taken as described elsewhere herein.

### II. Example Systems

An automated flow cytometry system (e.g., the iQue^{®} 3 or other systems of the ique^{®} series) may be used to generate, in an automated fashion, cell counts and/or bead counts, in addition to additional cytometric data (e.g., fluorescence emission spectra, light scatter, transmission spectra, Raman spectra, etc.), for a plurality of biological samples. These samples can be disposed within respective wells of a multi-well sample plate or other multi-sample container. The embodiments described herein can be employed by such an automated flow cytometry system to determine when to homogenize or resuspend the contents of a multi-well sample plate, to validate the accuracy of cell counts generated by the system, to adjust or otherwise correct cell counts detected by the system, to alert a user that an unacceptable level of settling and/or evaporation has occurred in the samples, to halt screening, or to provide some other benefit or functionality.

Use of such an automated flow cytometry system can significantly reduce the personnel costs of assessing biological samples, as well as increasing the consistency, with respect to timing, positioning, and volumetric parameters of the cell counts or other data generated when compared to manually presenting samples to a flow cytometry apparatus. Further, such an automated flow cytometry system can be configured to operate within an incubator or other controlled environment, removing the need to remove the samples from an incubator for assessment. Additionally, such an automated system can assess samples for extended periods of time and/or at inconvenient times (e.g., overnight), reducing technician costs and/or logistics. The technical advantages of a system operating in the manner described herein (to continuously assess in real time the level of settling and/or evaporation in biological samples by detecting the amount or concentration of one or more populations of indicator beads added to the samples and/or to neighboring sentinel wells) are related to the enhanced quality control of the screening platform via a more contemporaneous assessment of critical parameters such as cell counts, homogenous mixing, and remedial mixing to ensure cell health and accurate cell counts. This can increase user confidence, expose artifacts, and save money. It can also lead to enhanced clone selection.

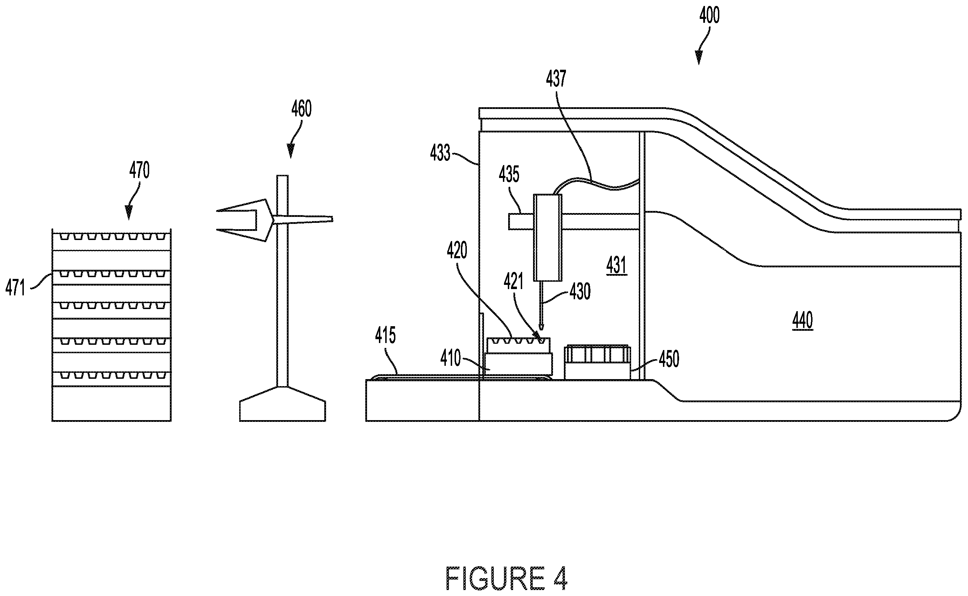

Figure 4 illustrates elements of such an automated flow cytometry system 400. The automated flow cytometry system 400 includes an enclosure 433 that defines a controlled operational volume 431 that is protected from particulates or other objects in the environment of the system 400. The enclosure 433 can be sealed (e.g., can include doors or hatches having sealable edges) so as to permit control of the environment within the operational volume 431 (e.g., temperature, humidity, light level). Alternatively, the enclosure 433 can have one or more openings (e.g., to facilitate movement of multi-well sample plates into and out of the operational volume 431).

A multi-well sample plate 420 that includes a plurality of sample wells (e.g., 421) is located within the operational volume 431 on a sample plate handler 410. The handler 410 is coupled to a belt 415 that can be operated to control the location of the handler 410 and any sample containers (e.g., the multi-well sample plate 420) disposed thereon. This can be done to move the handler 410 into and out of the enclosure 433, e.g., to facilitate the assessment of multiple sample containers by facilitating the loading and unloading of sample containers to/from the handler 410 outside of the enclosure 433. Additionally or alternatively, the belt 415 can be used to control the location of the handler 410 so as to facilitate assessment of the various individual samples of a multi-well sample plate 420 disposed thereon by sequentially aligning those sample wells (e.g., 421) with a needle or probe 430 of the flow cytometry system 400.

The probe 430 is also located within the operational volume 431. The probe 430 can be moved (e.g., by using a gantry 435 or other components) to sample from individual wells (e.g., well 421, with which is it horizontally aligned in Figure 4) so as to facilitate extracting volumes of fluid and cells disposed therein for flow cytometric analysis. The probe 430 can also be moved to sample from one or more fluid reservoirs 450 that are located within the operational volume 431. These reservoirs 450 can contain cleaning or wash fluids, reagents for use with the flow cytometer, calibration substances (e.g., substances containing a known number of beads per unit volume), or other fluids to facilitate the operation of the flow cytometry system 400. Fluids extracted through the probe 430 are then passed, via tubing, 437, to flow cells or other analytical apparatus located within a housing 440 of the flow cytometry system 400. Such analytical apparatus can include light emitters, light detectors, spectrometers, cameras, flow rate detectors, or other apparatus configured to detect a magnitude or spectrum of light passed through an extracted fluid, a magnitude or spectrum of light scattered off of cells or other contents of an extracted fluid, a flow rate of an extracted fluid, a density of an extracted fluid, a viscosity of an extracted fluid, an electrical impedance of an extracted fluid, or some other properties of a fluid extracted from a well or other sample-containing element (e.g., example well 421) of the multi-well sample plate 420 or of some other container configured to contain a plurality of separate samples.

Such detected physical properties can then be analyzed to determine cell types, absolute cell counts, cell counts normalized to sample volume, cell sizes, cell health, or other information about individual cells and/or about populations of cells, such as homogeneity of the cells, within a sample. Such analysis may be performed by controller(s) or other computing elements located within the housing 440. Additionally or altematively, some or all of such analyses can be performed by a server, computer, laptop, or other computing elements that are in communication with the automated flow cytometry system 400 so as to receive the detected physical properties (e.g., raw detected pass-through or scattered light intensities) and/or parameters determined therefrom (e.g., the type of each cell detected by the system 400 as determined from detected pass-through or scattered light intensities). Such communication can be accomplished via local communications links, e.g., Ethernet, Bluetooth, WiFi, USB, or other local links to a laptop, server, local network, or other computing device that is physically proximate to the system 400 (e.g., in the same room or building as the system 400). Additionally or alternatively, such communication can be accomplished via nonlocal links, e.g., the Intemet. This can allow portion of the computational analysis of a set of samples to be conducted remote from the system 400, e.g., in a researcher's office, in a server of a cloud computing environment, etc.

The analytical apparatus within the housing 440 can also include pumps, flow rate detection equipment, or other components to facilitate extraction of fluid from samples at a known and/or controlled volumetric flow rate. Such known/controllable flow rate sampling can improve flow cytometric analysis by facilitating the determination of cell populations in a sample in an absolute manner (e.g., an absolute count of cells in the sample, or a per-unit-volume count of cells in the sample), rather than relative to the population of other cells or other contents (e.g., calibration beads) in the sample. This can lead to reduced time to assess the samples by, e.g., removing additional analyses needed to relate a determined cell count to the volume of a sample. Additionally or altematively, this can lead to improved analyses by allowing calibration beads or other volume-detection methods to be omitted from the samples. Yet further, detecting cells counts in such a volume-controlled manner can provide a 'sanity check' for the cell counts, allowing for verification of the counts and increased confidence in the accuracy of the counts.

The actuated gantry 435 may include elements configured to facilitate detection and/or control of the absolute and/or relative location of the probe 430 relative to the multi-well sample plate 420 (e.g., to the example well 421 of the multi-well sample plate 420). For example, the actuated gantry 435 may include encoders, limit switches, and/or other location-sensing elements and/or stepper motors, servos, solenoids, or other location-controlling elements. In some examples, the system 400 can include a camera or other imaging apparatus to detect fiducial marks or other features of the multi-well sample plate 420 and/or of the probe 430 in order to determine the absolute and/or relative location of the probe 430 relative to the multi-well sample plate 420.

The multi-well sample plate 420 can include dozens, hundreds, or even thousands of individual sample-containing wells. For example, the multi-well sample plate 420 can have 96 or more wells, 384 or more wells, 1536 or more wells, or some larger number of wells. The multi-well sample plate 420 can comport with a standardized size or geometry for such multi-sample containers.

To increase the number of samples that the automated flow cytometry system 400 can assess with no or minimal human interventions, the system 400 can include a robotic arm 460. The robotic arm 460 includes servos or other actuators to facilitate moving multi-well sample plates (e.g., example plate 471) from a rack or tower 470 of such plates to the handler 410 (or some other sample plate receptacle) of the system 400. The robotic arm 460 can also operate to remove such sample plates from the handler 410 once they have been assessed (e.g., placing them back into their original position within the rack or tower 470, placing them in a "discard" rack or other location). The robotic arm 460 can be controlled directly by processors or other components of the automated flow cytometry system 400. Alternatively, the robotic arm 460 can include its own controller components and can operate semi-independently from the rest of the automated flow cytometry system 400, receiving commands or other signals from the automated flow cytometry system 400 or from some other controller system to indicate when to transfer sample plates to and/or from the handler 410.

Computational functions (e.g., functions to operate the actuated gantry 435, robot arm 460, and/or flow cytometric apparatus to assess volumes of fluid extracted from sample containers) may be performed by one or more computing systems. Such a computing system may be integrated into an automated flow cytometric system (e.g., 400), may be associated with such a system (e.g., by being connected via a direct wired or wireless connection, via a local network, and/or via a secured connection over the internet), and/or may take some other form (e.g., a cloud computing system that is in communication with an automated flow cytometry system and/or that has access to a store of physical properties detected thereby). Such a computing system may include a communication interface, a user interface, a processor, and data storage, all of which may be communicatively linked together by a system bus, network, or other connection mechanism.

The communication interface may function to allow the computing system to communicate, using analog or digital modulation of electric, magnetic, electromagnetic, optical, or other signals, with other devices, access networks, and/or transport networks. Thus, communication interface may facilitate circuit-switched and/or packet-switched communication, such as plain old telephone service (POTS) communication and/or Intemet protocol (IP) or other packetized communication. For instance, communication interface may include a chipset and antenna arranged for wireless communication with a radio access network or an access point. Also, communication interface may take the form of or include a wireline interface, such as an Ethernet, Universal Serial Bus (USB), or High-Definition Multimedia Interface (HDMI) port. Communication interface 402 may also take the form of or include a wireless interface, such as a WiFi, BLUETOOTH^{®}, global positioning system (GPS), or wide-area wireless interface (e.g., WiMAX or 3GPP Long-Term Evolution (LTE)). However, other forms of physical layer interfaces and other types of standard or proprietary communication protocols may be used over communication interface. Furthermore, communication interface may comprise multiple physical communication interfaces (e.g., a WiFi interface, a BLUETOOTH^{®} interface, and a wide-area wireless interface).

In some embodiments, the communication interface may function to allow computing system to communicate with other devices, remote servers, access networks, and/or transport networks. For example, the communication interface may function to transmit and/or receive an indication of cell counts or other information detected from biological samples (e.g., magnitudes and/or spectra of light transmitted through, reflected from, fluorescently emitted from, and/or scattered by cells of other contents in a sample) or some other information.

The user interface of such a computing system may function to allow computing system to interact with a user, for example to receive input from and/or to provide output to the user. Thus, user interface may include input components such as a keypad, keyboard, touch-sensitive or presence-sensitive panel, computer mouse, trackball, joystick, microphone, and so on. User interface may also include one or more output components such as a display screen which, for example, may be combined with a presence-sensitive panel. The display screen may be based on CRT, LCD, and/or LED technologies, or other technologies now known or later developed. User interface may also be configured to generate audible output(s), via a speaker, speaker jack, audio output port, audio output device, earphones, and/or other similar devices.

In some embodiments, the user interface may include a display that serves to present cell counts or other information about the sample to a user. Additionally, the user interface may include one or more buttons, switches, knobs, and/or dials that facilitate the configuration and operation of the computing device. It may be possible that some or all of these buttons, switches, knobs, and/or dials are implemented as functions on a touch- or presence-sensitive panel. The user interface may permit a user to specify the types of samples contained within an automated flow cytometry system, to specify flow rates or volumes for assessment of the samples, to specify characteristics of cells or calibration beads to facilitate detection thereof, or to input some other commands or parameters for operation of an automated flow cytometry system.

In some examples, portions of the methods described herein can be performed by different devices, according to an application. For example, different devices of a system can have different amounts of computational resources (e.g., memory, processor cycles) and different information bandwidths for communication between the devices. For example, a first device can be an embedded processor(s) that can operate an actuated gantry, pump, flow cytometry detector suite, or other elements to generate cell counts or other flow cytometric data for biological samples. A second device can then receive (e.g., via the internet, via a dedicated wired link), from the first device, the information from the first device and perform some additional processing and analysis on the received data. Different portions of the methods described herein can be apportioned according to such considerations.

### IV. Example Methods

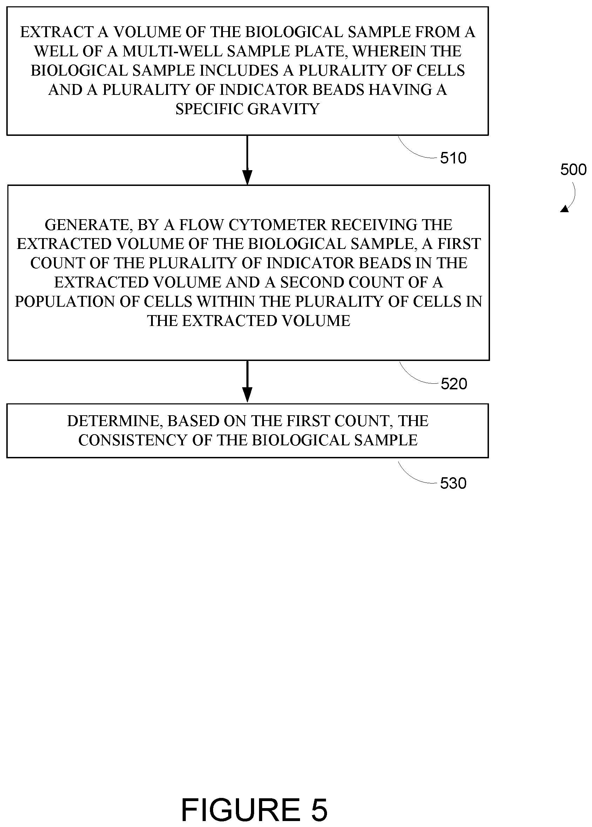

Figure 5 is a flowchart of a method 500 for detecting a consistency of a biological sample. The method 500 includes extracting a volume of the biological sample from a well of a multi-well sample plate, wherein the biological sample includes a plurality of cells and a plurality of indicator beads having a specific gravity (510). The method 500 additionally includes generating, by a flow cytometer receiving the extracted volume of the biological sample, a first count of the plurality of indicator beads in the extracted volume and a second count of a population of cells within the plurality of cells in the extracted volume (520). The method 500 additionally includes determining, based on the first count, the consistency of the biological sample (530). The method 500 can include additional or alternative steps or features.

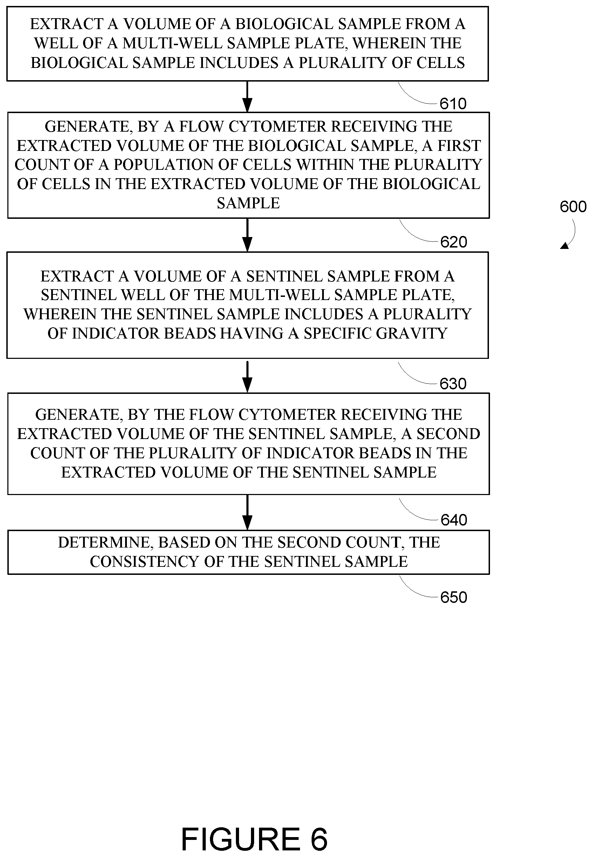

Figure 6 is a flowchart of a method 600 for detecting for detecting a consistency of a biological sample. The method 600 includes extracting a volume of a biological sample from a well of a multi-well sample plate, wherein the biological sample includes a plurality of cells (610). The method 600 additionally includes generating, by a flow cytometer receiving the extracted volume of the biological sample, a first count of a population of cells within the plurality of cells in the extracted volume of the biological sample (620). The method 600 additionally includes extracting a volume of a sentinel sample from a sentinel well of the multi-well sample plate, wherein the sentinel sample includes a plurality of indicator beads having a specific gravity (630). The method 600 additionally includes generating, by the flow cytometer receiving the extracted volume of the sentinel sample, a second count of the plurality of indicator beads in the extracted volume of the sentinel sample (640). The method 600 yet further includes determining, based on the second count, the consistency of the sentinel sample (650). The method 600 can include additional elements or features.

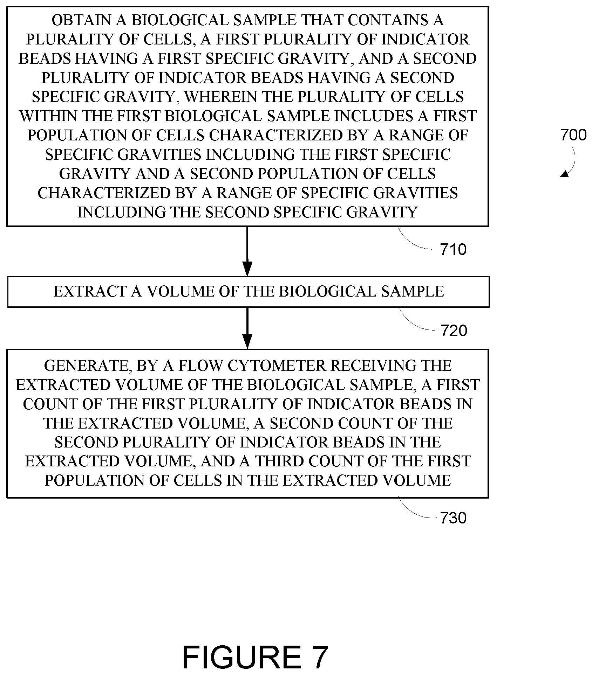

Figure 7 is a flowchart of a method 700 for detecting a consistency of a biological sample. The method 700 includes obtaining a biological sample that contains a plurality of cells, a first plurality of indicator beads having a first specific gravity, and a second plurality of indicator beads having a second specific gravity, wherein the plurality of cells within the first biological sample includes a first population of cells characterized by a range of specific gravities including the first specific gravity and a second population of cells characterized by a range of specific gravities including the second specific gravity (710). The method 700 additionally includes extracting a volume of the biological sample (720). The method 700 also includes generating, by a flow cytometer receiving the extracted volume of the biological sample, a first count of the first plurality of indicator beads in the extracted volume, a second count of the second plurality of indicator beads in the extracted volume, and a third count of the first population of cells in the extracted volume (730). The method 700 can include additional elements or features.

### V. Experimental Evaporation and Settling Data

It can take over an hour for the iQue^{®} 3 system to sample a full 1536-well plate. Experiments were performed to determine whether sufficient water would evaporate from the wells such that the biology would be adversely affected. To assess this, assay plates were filled with a measured amount of water and then placed in the iQue^{®} 3 system. The iQue^{®} system simulated sampling by moving the probe over, but not into, each of the wells. The probe did not come into contact with the water, so there was no water sampled from the wells. During the simulated sampling, a plate shaker was run according to pre-specified protocols to mimic resuspension of the sample within the wells. At regular intervals, the assay plates were then weighed to assess the amount of water remaining.

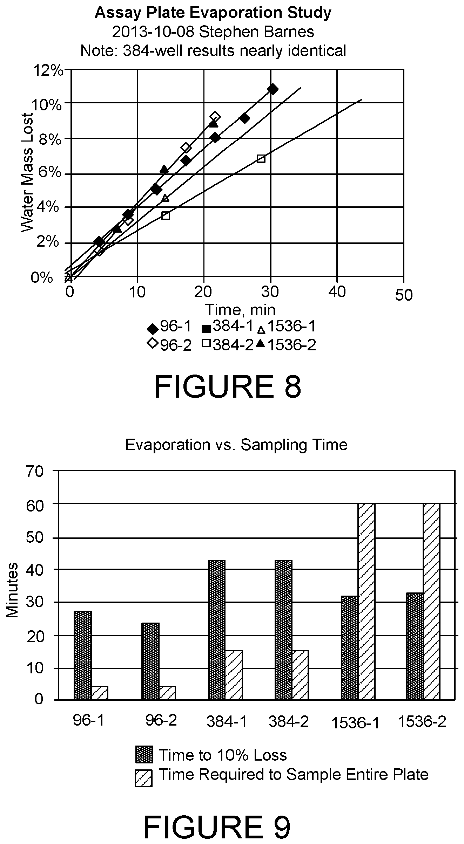

For repeatability, two plates of each of three types were tested. The 1536-well plates were filled using the Formulatrix Mantis. The 96 and 384-well plates were manually pipetted. The plates were covered during transportation and weighing. The simulated sampling was repeated as necessary until the plate lost near 10% of its original water mass. The 96-well plates were Model 10149, V-bottom, crystal plates, with a starting volume of 20 µL, a total water starting mass of 2.8 g, a simulated sampling time of 4 minutes 20 seconds, a sip time of 1 second, and a shaking protocol of 2400 RPM for 3 seconds after every 12 wells. The 384-well plates were Greiner 781280, V-bottom, polypropylene plates, with a starting volume of 15 µL, a total water starting mass of 5.6 g, a simulated sampling time of 15 minutes, a sip time of 1 second, and a shaking protocol of 3000 RPM for 3 seconds after every 24 wells. The 1536-well plates were Corning 3937, 10µl Round bottom, polystyrene plates, with a starting volume of 4 µL, a total water starting mass of 6.0 g, a simulated sampling time of 60 minutes, a sip time of 1 second, and a shaking protocol of 5000 RPM for 3 seconds after every 24 wells. Figure 8 shows the mass measurements relative to the original mass of the water in each plate. For the 96 and 384-well plates, the data points represent measurements that were completed after the entire plate was run. For the 1536-well plates, the data points represent measurements completed after only a portion of the plate was run (1/4 for 1536-1, 1/8 for 1536-2). It can be seen that the water lost from each plate occurred linearly with time. This may be due to the relative magnitude of the lost water being relatively small, that is, the surface area of the water in each well did not changing significantly. Also it can be seen that, on a relative basis, the different plate types lost water at roughly the same rate of 0.4% per minute. A biological assay can be adversely affected by losing 10% of its water. For example, Figure 9 shows, in blue, the time each plate took to lose 10% of its water. The time required to sample the entire plate is shown in red. Figure 9 further shows that the 96 and 384-well plates lost less than 10% of their water during sampling in the iQue^{®}, but the 1536-well plates lost more than 10% of their water during sampling. From this data and the 10% evaporation limit, it is concluded that sampling an open 1536-well plate in the iQue^{®} may exhibit issues related to water loss. In one hour of sampling, a 1536-well plate loses approximately 20% of its water. If the sampling takes 2 hours, it would likely lose 40%. In 15 minutes of sampling, a 384-well plate loses 4% of its water. In 4 minutes of sampling, a 96-well plate loses 1-2% of its water. Water was lost roughly linearly over time: The 96, 384, and 1536-well plates all lost 0.4% ± 0.2% of their water per minute. Further, the above testing was done in a lab having ambient conditions of 25°C and 26% relative humidity.

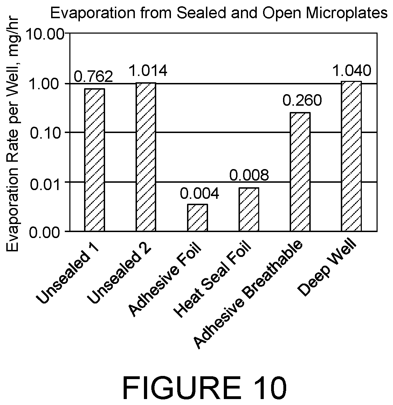

Similar experiments were also performed with 1536-well plates that had seals over the wells. The plates were Corning 3937 round well plates. Additional similar experiments with a 14 mm deep "deep well" plate from Greiner, part number 782270 were also performed to assess whether deeper wells can reduce evaporation. As shown in Figure 10, sealing the assay plates with foil seals (either adhesive or heat seal) reduced the evaporation to negligible amounts. Putting a breathable seal on the plate reduced evaporation by 71%. Further, the deep well Greiner plate showed no significant reduction in evaporation. The beads described above, and related methods for counting to assess the consistency of the samples, could be applied in addition to these capping means in order to evaluate the effectiveness of the capping means.

### VII. Conclusion

With respect to any or all of the message flow diagrams, scenarios, and flowcharts in the figures and as discussed herein, each step, block and/or communication may represent a processing of information and/or a transmission of information in accordance with example embodiments. Alternative embodiments are included within the scope of these example embodiments. In these alternative embodiments, for example, functions described as steps, blocks, transmissions, communications, requests, responses, and/or messages may be executed out of order from that shown or discussed, including in substantially concurrent or in reverse order, depending on the functionality involved. Further, more or fewer steps, blocks and/or functions may be used with any of the message flow diagrams, scenarios, and flow charts discussed herein, and these message flow diagrams, scenarios, and flow charts may be combined with one another, in part or in whole.

A step or block that represents a processing of information may correspond to circuitry that can be configured to perform the specific logical functions of a herein-described method or technique. Alternatively or additionally, a step or block that represents a processing of information may correspond to a module, a segment, or a portion of program code (including related data). The program code may include one or more instructions executable by a processor for implementing specific logical functions or actions in the method or technique. The program code and/or related data may be stored on any type of computer-readable medium, such as a storage device, including a disk drive, a hard drive, or other storage media.

The computer-readable medium may also include non-transitory computer-readable media such as computer-readable media that stores data for short periods of time like register memory, processor cache, and/or random-access memory (RAM). The computer-readable media may also include non-transitory computer-readable media that stores program code and/or data for longer periods of time, such as secondary or persistent long term storage, like read only memory (ROM), optical or magnetic disks, and/or compact-disc read only memory (CD-ROM), for example. The computer-readable media may also be any other volatile or non-volatile storage systems. A computer-readable medium may be considered a computer-readable storage medium, for example, or a tangible storage device.

Moreover, a step or block that represents one or more information transmissions may correspond to information transmissions between software and/or hardware modules in the same physical device. However, other information transmissions may be between software modules and/or hardware modules in different physical devices.

While various aspects and embodiments have been disclosed herein, other aspects and embodiments will be apparent to those skilled in the art without departing from the scope of the invention as defined in the appended claims.

## Claims

1. A method for detecting a consistency of a biological sample (110), the method comprising:
extracting a volume of the biological sample (110) from a well (421) of a multi-well sample plate (420), wherein the biological sample (110) includes a plurality of cells and a plurality of indicator beads having a specific gravity;
generating, by a flow cytometer (400) receiving the extracted volume of the biological sample, a first count of the plurality of indicator beads in the extracted volume and a second count of a population of cells within the plurality of cells in the extracted volume (520); and
determining, based on the first count, the consistency of the biological sample (530) which comprises determining that an observed concentration of the plurality of indicator beads deviates from an expected concentration of the plurality of indicator beads in the biological sample,
**characterized in that** the method further comprises:
determining that the consistency of the biological sample deviates from an expected consistency by more than a threshold value; and
responsively resuspending the plurality of cells in the biological sample in the multi-well sample plate (420).

2. The method of claim 1, wherein the multi-well sample plate (420) includes 96 wells, 384 wells, or 1536 wells (421).

3. The method of claim 1 or claim 2, wherein the plurality of indicator beads included in the biological sample includes a first plurality of indicator beads having a first specific gravity and a second plurality of indicator beads having a second specific gravity,
wherein the method further comprises:
generating, by the flow cytometer (400) receiving the extracted volume (520) of the biological sample, the first count of the first plurality of indicator beads, the second count of the population of cells in the extracted volume, and a third count of the second plurality of indicator beads, and
wherein determining, based on the first count, the consistency of the biological sample comprises:
determining, based on the first count and the third count, that an observed ratio between the first plurality of indicator beads and the second plurality of indicator beads deviates from an expected ratio between the first plurality of indicator beads and the second plurality of indicator beads in the biological sample.

4. The method of claim 3, wherein the plurality of cells within the biological sample are **characterized by** a range of specific gravities, including a first population of cells **characterized by** a range of specific gravities including the first specific gravity and a second population of cells **characterized by** a range of specific gravities including the second specific gravity.

5. The method of any preceding claim, further comprising:
based on the consistency of the biological sample, correcting the second count of the population of cells in the biological sample.

6. The method of claim 5, wherein correcting the second count comprises determining a ratio between the first count and an expected count of the plurality of indicator beads in the extracted volume and scaling the second count based on the determined ratio.

7. The method of any preceding claim,
when determining that the consistency of the biological sample deviates from an expected consistency by more than a threshold value; further comprising:
responsively adding an annotation to a record that is associated with the biological sample that includes the second count of the population of cells in the biological sample.

8. The method of any preceding claim,
when determining that the consistency of the biological sample deviates from an expected consistency by more than a threshold value; further comprising:
responsively providing, via a user interface, an alert.

9. The method of any preceding claim, further comprising:
subsequent to resuspending the plurality of cells in the biological sample, extracting a volume of a second biological sample from a well (421) of the multi-well sample plate (420) and generating, by the flow cytometer (400) receiving the extracted volume of the second biological sample, at least one count of at least one population of cells within the extracted volume of the second biological sample.

10. The method of claim 9, further comprising:
generating, by the flow cytometer (400) receiving the extracted volume of the second biological sample, a count of the plurality of indicator beads within the extracted volume of the second biological sample; and
determining, based on the count of the plurality of indicator beads within the extracted volume of the second biological sample, the consistency of the biological sample subsequent to resuspending the plurality of cells in the biological sample.

11. The method of claim 10, wherein the volume of the second biological sample is extracted from the same well of the multi-well sample plate (420) as the well from which the volume used to generate the first and second counts was extracted, and wherein the method further comprises:
determining that the consistency of the biological sample subsequent to resuspending the plurality of cells in the biological sample deviates from an expected consistency by less than a threshold value; and
responsively extracting a volume of a third biological sample from a different well of the multi-well sample plate (420) and generating, by the flow cytometer (400) receiving the extracted volume of the third biological sample, at least one count of at least one population of cells within the extracted volume of the third biological sample.

12. The method of any preceding claim, wherein resuspending the plurality of cells in the multi-well sample plate (420) comprises at least one of shaking the multi-well sample plate (420), using a probe to stir the contents of the multi-well sample plate (420), or repeatedly aspirating fluid from the multi-well sample plate (420).

13. A non-transitory computer-readable medium, configured to store at least computer-readable instructions that, when executed by one or more processors of a computing device, cause the computing device to perform computer operations to cause a system for detecting a consistency of a biological sample to carry out the method of any of claims 1-12,
wherein the system comprises:
- means configured to perform the steps of
extracting a volume of the biological sample (110) from a well (421) of a multi-well sample plate (420), wherein the biological sample (110) includes a plurality of cells and a plurality of indicator beads having a specific gravity; and
responsively resuspending the plurality of cells in the biological sample in the multi-well sample plate (420)
of claim 1;
- a flow cytometer configured to perform the step of
generating, by a flow cytometer (400) receiving the extracted volume of the biological sample, a first count of the plurality of indicator beads in the extracted volume and a second count of a population of cells within the plurality of cells in the extracted volume (520)
of claim 1; and
- one or more processors configured to perform the steps of
determining, based on the first count, the consistency of the biological sample (530) which comprises determining that an observed concentration of the plurality of indicator beads deviates from an expected concentration of the plurality of indicator beads in the biological sample; and
determining that the consistency of the biological sample deviates from an expected consistency by more than a threshold value
of claim 1.

14. A system for detecting a consistency of a biological sample, the system comprising:
one or more processors; and
a non-transitory computer-readable medium, configured to store at least computer-readable instructions that, when executed by the one or more processors, cause the system to perform the method of any of claims 1-12,
wherein the system further comprises:
- means configured to perform the steps of
extracting a volume of the biological sample (110) from a well (421) of a multi-well sample plate (420), wherein the biological sample (110) includes a plurality of cells and a plurality of indicator beads having a specific gravity; and
responsively resuspending the plurality of cells in the biological sample in the multi-well sample plate (420)
of claim 1;
- a flow cytometer configured to perform the step of
generating, by a flow cytometer (400) receiving the extracted volume of the biological sample, a first count of the plurality of indicator beads in the extracted volume and a second count of a population of cells within the plurality of cells in the extracted volume (520)
of claim 1; and
- one or more processors configured to perform the steps of
determining, based on the first count, the consistency of the biological sample (530) which comprises determining that an observed concentration of the plurality of indicator beads deviates from an expected concentration of the plurality of indicator beads in the biological sample; and
determining that the consistency of the biological sample deviates from an expected consistency by more than a threshold value
of claim 1.

## Patentansprüche

1. Verfahren zum Feststellen einer Konsistenz einer biologischen Probe (110), wobei das Verfahren umfasst:
Entnehmen eines Volumens der biologischen Probe (110) von einem Well (421) einer Mehrfach-Well-Probenplatte (420), wobei die biologische Probe (110) eine Vielzahl von Zellen und eine Vielzahl von Indikator-Kügelchen umfasst, die ein spezifisches Gewicht aufweisen;
Erzeugen, durch ein Durchflusszytometer (400), welches das entnommene Volumen der biologischen Probe aufnimmt, einer ersten Zählung der Vielzahl von Indikator-Kügelchen in dem entnommenen Volumen und einer zweiten Zählung einer Population von Zellen innerhalb der Vielzahl von Zellen in dem entnommenen Volumen (520); und
Bestimmen, auf der Grundlage der ersten Zählung, der Konsistenz der biologischen Probe (530), welches das Bestimmen umfasst, dass eine beobachtete Konzentration der Vielzahl von Indikator-Kügelchen von einer erwarteten Konzentration der Vielzahl von Indikator-Kügelchen in der biologischen Probe abweicht,
**dadurch gekennzeichnet, dass** das Verfahren ferner umfasst:
Bestimmen, dass die Konsistenz der biologischen Probe um mehr als ein Schwellenwert von einer erwarteten Konsistenz abweicht; und
als Reaktion darauf, Resuspendieren der Vielzahl von Zellen in der biologischen Probe in der Mehrfach-Well-Probenplatte (420).

2. Verfahren nach Anspruch 1, wobei die Mehrfach-Well-Probenplatte (420) 96 Wells, 384 Wells oder 1536 Wells (421) umfasst.

3. Verfahren nach Anspruch 1 oder Anspruch 2, wobei die Vielzahl von Indikator-Kügelchen, die in der biologischen Probe enthalten sind, eine erste Vielzahl von Indikator-Kügelchen, die ein erstes spezifisches Gewicht aufweisen, und eine zweite Vielzahl von Indikator-Kügelchen umfassen, die ein zweites spezifisches Gewicht aufweisen,
wobei das Verfahren ferner umfasst:
Erzeugen, durch das Durchflusszytometer (400), welches das entnommene Volumen (520) der biologischen Probe aufnimmt, der ersten Zählung der ersten Vielzahl von Indikator-Kügelchen, der zweiten Zählung der Population von Zellen in dem entnommenen Volumen und einer dritten Zählung der zweiten Vielzahl von Indikator-Kügelchen, und
wobei das Bestimmen der Konsistenz der biologischen Probe auf der Grundlage der ersten Zählung umfasst:
Bestimmen, auf der Grundlage der ersten Zählung und der dritten Zählung, dass ein beobachtetes Verhältnis zwischen der ersten Vielzahl von Indikator-Kügelchen und der zweiten Vielzahl von Indikator-Kügelchen von einem erwarteten Verhältnis zwischen der ersten Vielzahl von Indikator-Kügelchen und der zweiten Vielzahl von Indikator-Kügelchen in der biologischen Probe abweicht.

4. Verfahren nach Anspruch 3, wobei die Vielzahl von Zellen innerhalb der biologischen Probe durch einen Bereich von spezifischen Gewichten gekennzeichnet sind, umfassend eine erste Population von Zellen, die durch einen Bereich von spezifischen Gewichten gekennzeichnet sind, der das erste spezifische Gewicht umfasst, und eine zweite Population von Zellen, die durch einen Bereich von spezifischen Gewichten gekennzeichnet sind, der das zweite spezifische Gewicht umfasst.

5. Verfahren nach einem der vorhergehenden Ansprüche, ferner umfassend:
auf der Grundlage der Konsistenz der biologischen Probe, Korrigieren der zweiten Zählung der Population von Zellen in der biologischen Probe.

6. Verfahren nach Anspruch 5, wobei das Korrigieren der zweiten Zählung das Bestimmen eines Verhältnisses zwischen der ersten Zählung und einer erwarteten Zählung der Vielzahl von Indikator-Kügelchen in dem entnommenen Volumen und das Skalieren der zweiten Zählung auf der Grundlage des bestimmten Verhältnisses umfasst.

7. Verfahren nach einem der vorhergehenden Ansprüche, wobei das Bestimmen, dass die Konsistenz der biologischen Probe um mehr als ein Schwellenwert von einer erwarteten Konsistenz abweicht, ferner umfasst:
als Reaktion darauf, Hinzufügen eines Vermerks zu einem Datensatz, der mit der biologischen Probe verbunden ist, welche die zweite Zählung der Population von Zellen in der biologischen Probe umfasst.

8. Verfahren nach einem der vorhergehenden Ansprüche,
wobei das Bestimmen, dass die Konsistenz der biologischen Probe um mehr als ein Schwellenwert von einer erwarteten Konsistenz abweicht, ferner umfasst:
als Reaktion darauf, Bereitstellen eines Alarms über eine Benutzerschnittstelle.

9. Verfahren nach einem der vorhergehenden Ansprüche, ferner umfassend:
im Anschluss an das Resuspendieren der Vielzahl von Zellen in der biologischen Probe, Entnehmen eines Volumens einer zweiten biologischen Probe von einem Well (421) der Mehrfach-Well-Probenplatte (420) und Erzeugen, durch das Durchflusszytometer (400), welches das entnommene Volumen der zweiten biologischen Probe aufnimmt, mindestens einer Zählung von mindestens einer Population von Zellen innerhalb des entnommenen Volumens der zweiten biologischen Probe.

10. Verfahren nach Anspruch 9, ferner umfassend:
Erzeugen, durch das Durchflusszytometer (400), welches das entnommene Volumen der zweiten biologischen Probe aufnimmt, einer Zählung der Vielzahl von Indikator-Kügelchen innerhalb des entnommenen Volumens der zweiten biologischen Probe; und
Bestimmen, auf der Grundlage der Zählung der Vielzahl von Indikator-Kügelchen innerhalb des entnommenen Volumens der zweiten biologischen Probe, der Konsistenz der biologischen Probe im Anschluss an das Resuspendieren der Vielzahl von Zellen in der biologischen Probe.

11. Verfahren nach Anspruch 10, wobei das Volumen der zweiten biologischen Probe von demselben Well der Mehrfach-Well-Probenplatte (420) entnommen wird wie der Well, von dem das Volumen entnommen wurde, das zum Erzeugen der ersten und der zweiten Zählung verwendet wurde, und wobei das Verfahren ferner umfasst:
Bestimmen, dass die Konsistenz der biologischen Probe im Anschluss an das Resuspendieren der Vielzahl von Zellen in der biologischen Probe um weniger als ein Schwellenwert von einer erwarteten Konsistenz abweicht; und
als Reaktion darauf, Entnehmen eines Volumens einer dritten biologischen Probe von einem unterschiedlichen Well der Mehrfach-Well-Probenplatte (420) und Erzeugen, durch das Durchflusszytometer (400), welches das entnommene Volumen der dritten biologischen Probe aufnimmt, mindestens einer Zählung von mindestens einer Population von Zellen innerhalb des entnommenen Volumens der dritten biologischen Probe.

12. Verfahren nach einem der vorhergehenden Ansprüche, wobei das Resuspendieren der Vielzahl von Zellen in der Mehrfach-Well-Probenplatte (420) mindestens eines von Schütteln der Mehrfach-Well-Probenplatte (420), Verwenden einer Sonde zum Rühren der Inhalte der Mehrfach-Well-Probenplatte (420) oder wiederholtem Ansaugen von Fluid von der Mehrfach-Well-Probenplatte (420) umfasst.

13. Nichtflüchtiger maschinenlesbarer Datenträger, der dazu ausgestaltet ist, mindestens maschinenlesbare Anweisungen zu speichern, die, wenn sie von einem oder mehreren Prozessoren einer Rechenvorrichtung ausgeführt werden, die Rechenvorrichtung veranlassen, Rechneroperationen durchzuführen, um ein System zum Feststellen einer Konsistenz einer biologischen Probe zu veranlassen, das Verfahren nach einem der Ansprüche 1 bis 12 durchzuführen,
wobei das System umfasst:
- Mittel, die dazu ausgestaltet sind, die Schritte zum
Entnehmen eines Volumens der biologischen Probe (110) von einem Well (421) einer Mehrfach-Well-Probenplatte (420), wobei die biologische Probe (110) eine Vielzahl von Zellen und eine Vielzahl von Indikator-Kügelchen umfasst, die ein spezifisches Gewicht aufweisen; und
als Reaktion darauf, Resuspendieren der Vielzahl von Zellen in der biologischen Probe in der Mehrfach-Well-Probenplatte (420)
nach Anspruch 1 durchzuführen;
- ein Durchflusszytometer, das dazu ausgestaltet ist, den Schritt zum
Erzeugen, durch ein Durchflusszytometer (400), welches das entnommene Volumen der biologischen Probe aufnimmt, einer ersten Zählung der Vielzahl von Indikator-Kügelchen in dem entnommenen Volumen und einer zweiten Zählung einer Population von Zellen innerhalb der Vielzahl von Zellen in dem entnommenen Volumen (520) nach Anspruch 1 durchzuführen; und
- einen oder mehrere Prozessoren, der bzw. die dazu ausgestaltet ist bzw. sind, die Schritte zum
Bestimmen, auf der Grundlage der ersten Zählung, der Konsistenz der biologischen Probe (530), welches das Bestimmen umfasst, dass eine beobachtete Konzentration der Vielzahl von Indikator-Kügelchen von einer erwarteten Konzentration der Vielzahl von Indikator-Kügelchen in der biologischen Probe abweicht; und
Bestimmen, dass die Konsistenz der biologischen Probe um mehr als ein Schwellenwert von einer erwarteten Konsistenz abweicht,
nach Anspruch 1 durchzuführen.

14. System zum Feststellen einer Konsistenz einer biologischen Probe, wobei das System umfasst:
einen oder mehrere Prozessoren; und
einen nichtflüchtigen maschinenlesbaren Datenträger, der dazu ausgestaltet ist, mindestens maschinenlesbare Anweisungen zu speichern, die, wenn sie von dem einen oder den mehreren Prozessoren ausgeführt werden, das System veranlassen, das Verfahren nach einem der Ansprüche 1 bis 12 durchzuführen,
wobei das System ferner umfasst:
- Mittel, die dazu ausgestaltet sind, die Schritte zum
Entnehmen eines Volumens der biologischen Probe (110) von einem Well (421) einer Mehrfach-Well-Probenplatte (420), wobei die biologische Probe (110) eine Vielzahl von Zellen und eine Vielzahl von Indikator-Kügelchen umfasst, die ein spezifisches Gewicht aufweisen; und
als Reaktion darauf, Resuspendieren der Vielzahl von Zellen in der biologischen Probe in der Mehrfach-Well-Probenplatte (420)
nach Anspruch 1 durchzuführen;
- ein Durchflusszytometer, das dazu ausgestaltet ist, den Schritt zum
Erzeugen, durch ein Durchflusszytometer (400), welches das entnommene Volumen der biologischen Probe aufnimmt, einer ersten Zählung der Vielzahl von Indikator-Kügelchen in dem entnommenen Volumen und einer zweiten Zählung einer Population von Zellen innerhalb der Vielzahl von Zellen in dem entnommenen Volumen (520)
nach Anspruch 1 durchzuführen; und
- einen oder mehrere Prozessoren, der bzw. die dazu ausgestaltet ist bzw. sind, die Schritte zum
Bestimmen, auf der Grundlage der ersten Zählung, der Konsistenz der biologischen Probe (530), welches das Bestimmen umfasst, dass eine beobachtete Konzentration der Vielzahl von Indikator-Kügelchen von einer erwarteten Konzentration der Vielzahl von Indikator-Kügelchen in der biologischen Probe abweicht; und
Bestimmen, dass die Konsistenz der biologischen Probe um mehr als ein Schwellenwert von einer erwarteten Konsistenz abweicht,
nach Anspruch 1 durchzuführen.

## Revendications

1. Procédé pour détecter la cohérence d'un échantillon biologique (110), le procédé comprenant :
l'extraction d'un volume de l'échantillon biologique (110) depuis un puits (421) d'une plaque d'échantillons à puits multiples (420), dans laquelle l'échantillon biologique (110) contient une pluralité de cellules et une pluralité de perles indicatrices ayant une certaine densité relative ;
la génération, par un cytomètre en flux (400) recevant le volume extrait de l'échantillon biologique, d'un premier décompte de la pluralité de perles indicatrices dans le volume extrait et d'un deuxième décompte d'une population de cellules parmi la pluralité de cellules dans le volume extrait (520) ; et
la détermination, sur la base du premier décompte, de la cohérence de l'échantillon biologique (530), qui comprend la détermination qu'une concentration observée de la pluralité de perles indicatrices s'écarte d'une concentration prévue de la pluralité de perles indicatrices dans l'échantillon biologique,
**caractérisé en ce que** le procédé comprend en outre :
la détermination que la cohérence de l'échantillon biologique s'écarte d'une cohérence prévue d'une valeur supérieure à une valeur seuil ; et
en réponse, la remise en suspension de la pluralité de cellules dans l'échantillon biologique dans la plaque d'échantillons à puits multiples (420).

2. Procédé selon la revendication 1, dans lequel la plaque d'échantillons à puits multiples (420) comprend 96 puits, 384 puits ou 1536 puits (421).

3. Procédé selon la revendication 1 ou la revendication 2, dans lequel la pluralité de perles indicatrices incluses dans l'échantillon biologique comprend une première pluralité de perles indicatrices ayant une première densité relative et une deuxième pluralité de perles indicatrices ayant une deuxième densité relative,
lequel procédé comprend en outre :
la génération, par le cytomètre en flux (400) recevant le volume extrait (520) de l'échantillon biologique, du premier décompte de la première pluralité de perles indicatrices, du deuxième décompte de la population de cellules dans le volume extrait, et d'un troisième décompte de la deuxième pluralité de perles indicatrices, et
dans lequel la détermination, sur la base du premier décompte, de la cohérence de l'échantillon biologique comprend :
la détermination, sur la base du premier décompte et du troisième décompte, qu'un rapport observé entre la première pluralité de perles indicatrices et la deuxième pluralité de perles indicatrices s'écarte d'un rapport prévu entre la première pluralité de perles indicatrices et la deuxième pluralité de perles indicatrices dans l'échantillon biologique.

4. Procédé selon la revendication 3, dans lequel la pluralité de cellules à l'intérieur de l'échantillon biologique est **caractérisée par** une plage de densités relatives, comprenant une première population de cellules **caractérisée par** une plage de densités relatives incluant la première densité relative et une deuxième population de cellules **caractérisée par** une plage de densités relatives incluant la deuxième densité relative.

5. Procédé selon l'une quelconque des revendications précédentes, comprenant en outre :
sur la base de la cohérence de l'échantillon biologique, la correction du deuxième décompte de la population de cellules dans l'échantillon biologique.

6. Procédé selon la revendication 5, dans lequel la correction du deuxième décompte comprend la détermination du rapport entre le premier décompte et un décompte prévu de la pluralité de perles indicatrices dans le volume extrait et la mise à l'échelle du deuxième décompte sur la base du rapport déterminé.

7. Procédé selon l'une quelconque des revendications précédentes,
lors de la détermination que la cohérence de l'échantillon biologique s'écarte d'une cohérence prévue d'une valeur supérieure à une valeur seuil, comprenant en outre :
l'addition en réponse d'une annotation à un enregistrement qui est associé à l'échantillon biologique qui inclut le deuxième décompte de la population de cellules dans l'échantillon biologique.

8. Procédé selon l'une quelconque des revendications précédentes,
lors de la détermination que la cohérence de l'échantillon biologique s'écarte d'une cohérence prévue d'une valeur supérieure à une valeur seuil, comprenant en outre :
la fourniture en réponse, via une interface utilisateur, d'une alerte.

9. Procédé selon l'une quelconque des revendications précédentes, comprenant en outre :
suite à la remise en suspension de la pluralité de cellules dans l'échantillon biologique,
l'extraction d'un volume d'un deuxième échantillon biologique depuis un puits (421) de la plaque d'échantillons à puits multiples (420) et la génération, par le cytomètre en flux (400) recevant le volume extrait du deuxième échantillon biologique, d'au moins un décompte d'au moins une population de cellules à l'intérieur du volume extrait du deuxième échantillon biologique.

10. Procédé selon la revendication 9, comprenant en outre :
la génération, par le cytomètre en flux (400) recevant le volume extrait du deuxième échantillon biologique, d'un décompte de la pluralité de perles indicatrices à l'intérieur du volume extrait du deuxième échantillon biologique ; et
la détermination, sur la base du décompte de la pluralité de perles indicatrices à l'intérieur du volume extrait du deuxième échantillon biologique, de la cohérence de l'échantillon biologique suite à la remise en suspension de la pluralité de cellules dans l'échantillon biologique.

11. Procédé selon la revendication 10, dans lequel le volume du deuxième échantillon biologique est extrait depuis le même puits de la plaque d'échantillons à puits multiples (420) que le puits depuis lequel le volume utilisé pour générer les premier et deuxième décomptes a été extrait, et lequel procédé comprend en outre :
la détermination que la cohérence de l'échantillon biologique suite à la remise en suspension de la pluralité de cellules dans l'échantillon biologique s'écarte d'une cohérence prévue d'une valeur inférieure à une valeur seuil ; et
en réponse l'extraction d'un volume d'un troisième échantillon biologique depuis un puits différent de la plaque d'échantillons à puits multiples (420) et la génération, par le cytomètre en flux (400) recevant le volume extrait du troisième échantillon biologique, d'au moins un décompte d'au moins une population de cellules à l'intérieur du volume extrait du troisième échantillon biologique.

12. Procédé selon l'une quelconque des revendications précédentes, dans lequel la remise en suspension de la pluralité de cellules dans la plaque d'échantillons à puits multiples (420) comprend au moins l'un parmi le secouage de la plaque d'échantillons à puits multiples (420), l'utilisation d'une sonde pour agiter les contenus de la plaque d'échantillon à puits multiples (420), et l'aspiration répétée de fluide à partir de la plaque d'échantillons à puits multiples (420).

13. Support non transitoire lisible par ordinateur, configuré pour stocker au moins des instructions lisibles par ordinateur qui, lorsqu'elles sont exécutées par un ou plusieurs processeurs d'un dispositif informatique, amènent le dispositif informatique à effectuer des opérations de calcul pour amener le système à détecter la cohérence d'un échantillon biologique pour mettre en œuvre le procédé de l'une quelconque des revendications 1 à 12,
dans lequel le système comprend :
- des moyens configurés pour effectuer les étapes de
extraction d'un volume de l'échantillon biologique (110) depuis un puits (421) d'une plaque d'échantillons à puits multiples (420), dans laquelle l'échantillon biologique (110) contient une pluralité de cellules et une pluralité de perles indicatrices ayant une certaine densité relative ; et
en réponse, remise en suspension de la pluralité de cellules dans l'échantillon biologique dans la plaque d'échantillons à puits multiples (420)
selon la revendication 1 ;
- un cytomètre en flux configuré pour effectuer l'étape de
génération, par un cytomètre en flux (400) recevant le volume extrait de l'échantillon biologique, d'un premier décompte de la pluralité de perles indicatrices dans le volume extrait et d'un deuxième décompte d'une population de cellules parmi la pluralité de cellules dans le volume extrait (520)
selon la revendication 1 ; et
- un ou plusieurs processeurs configurés pour effectuer les étapes de
détermination, sur la base du premier décompte, de la cohérence de l'échantillon biologique (530), qui comprend la détermination qu'une concentration observée de la pluralité de perles indicatrices s'écarte d'une concentration prévue de la pluralité de perles indicatrices dans l'échantillon biologique ; et
détermination que la cohérence de l'échantillon biologique s'écarte d'une cohérence prévue d'une valeur supérieure à une valeur seuil
selon la revendication 1.

14. Système pour détecter la cohérence d'un échantillon biologique, le système comprenant :
un ou plusieurs processeurs ; et
un support non transitoire lisible par ordinateur, configuré pour stocker au moins des instructions lisibles par ordinateur qui, lorsqu'elles sont exécutées le ou les processeurs, amènent le système à mettre en œuvre le procédé de l'une quelconque des revendications 1 à 12,
lequel système comprend en outre :
- des moyens configurés pour effectuer les étapes de
extraction d'un volume de l'échantillon biologique (110) depuis un puits (421) d'une plaque d'échantillons à puits multiples (420), dans laquelle l'échantillon biologique (110) contient une pluralité de cellules et une pluralité de perles indicatrices ayant une certaine densité relative ; et
en réponse, remise en suspension de la pluralité de cellules dans l'échantillon biologique dans la plaque d'échantillons à puits multiples (420)
selon la revendication 1 ;
- un cytomètre en flux configuré pour effectuer l'étape de
génération, par un cytomètre en flux (400) recevant le volume extrait de l'échantillon biologique, d'un premier décompte de la pluralité de perles indicatrices dans le volume extrait et d'un deuxième décompte d'une population de cellules parmi la pluralité de cellules dans le volume extrait (520)
selon la revendication 1 ; et
- un ou plusieurs processeurs configurés pour effectuer les étapes de
détermination, sur la base du premier décompte, de la cohérence de l'échantillon biologique (530), qui comprend la détermination qu'une concentration observée de la pluralité de perles indicatrices s'écarte d'une concentration prévue de la pluralité de perles indicatrices dans l'échantillon biologique ; et
détermination que la cohérence de l'échantillon biologique s'écarte d'une cohérence prévue d'une valeur supérieure à une valeur seuil
selon la revendication 1.
